(19) Europäisches Patentamt / European Patent Office / Office européen des brevets

(11) **EP 4 176 866 B1**

(12) **EUROPÄISCHE PATENTSCHRIFT**

(45) Veröffentlichungstag und Bekanntmachung des Hinweises auf die Patenterteilung:
**14.08.2024 Patentblatt 2024/33**

(21) Anmeldenummer: **22200308.9**

(22) Anmeldetag: **07.10.2022**

(51) Internationale Patentklassifikation (IPC):
***A61K 8/73*** *(2006.01)* ***A61Q 5/06*** *(2006.01)*
***A61K 8/34*** *(2006.01)* ***A61K 8/81*** *(2006.01)*

(52) Gemeinsame Patentklassifikation (CPC):
**A61K 8/732; A61K 8/34; A61K 8/8135; A61Q 5/06;**
A61K 2800/594

(54) **KOSMETISCHE MITTEL ZUR TEMPORÄREN UMFORMUNG VON KERATINISCHEN FASERN MIT STÄRKEDERIVATEN UND HOHEM LANGZEITHALT**

COSMETIC AGENTS FOR TEMPORARILY SHAPING KERATIN FIBERS, CONTAINING STARCH DERIVATIVES AND HAVING A HIGH LONGEVITY

PRODUITS COSMÉTIQUES POUR LA MISE EN FORME TEMPORAIRE DE FIBRES KÉRATINIQUES COMPRENANT DES DÉRIVÉS D’AMIDON ET UN MAINTIEN ÉLEVÉ À LONG TERME

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **09.11.2021 DE 102021212563**

(43) Veröffentlichungstag der Anmeldung:
**10.05.2023 Patentblatt 2023/19**

(73) Patentinhaber: **Henkel AG & Co. KGaA**
**40589 Düsseldorf (DE)**

(72) Erfinder:
- **Martinez, Cyrielle**
  **22549 Hamburg (DE)**
- **Metten, Diane**
  **22393 Hamburg (DE)**

(56) Entgegenhaltungen:
**EP-B1- 1 395 234**
**WO-A1-2017/109182**
**CN-A- 103 607 994**
**US-A1- 2006 182 702**
**US-A1- 2008 031 841**
**US-B2- 10 517 811**

EP 4 176 866 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft ein kosmetisches Mittel zur temporären Umformung von keratinischen Fasern, insbesondere menschlichen Haaren.

Die temporäre Gestaltung von Frisuren für einen längeren Zeitraum bis hin zu mehreren Tagen erfordert in der Regel die Anwendung festigender Wirkstoffe. Daher spielen Haarbehandlungsmittel, die einer temporären Formgebung der Haare dienen, eine wichtige Rolle. Entsprechende Mittel zur temporären Verformung enthalten als festigenden Wirkstoff üblicherweise synthetische Polymere und/oder Wachse. Mittel zur Unterstützung der temporären Umformung von Haaren können beispielsweise als Haarspray, Haarwachs, Haargel, Haarschaum konfektioniert werden.

Eine wichtige Eigenschaft eines Mittels zur temporären Verformung von Haaren, im Folgenden auch Stylingmittel genannt, besteht darin, den behandelten Fasern in der neu modellierten Form - das heißt einer den Haaren aufgeprägten Form - einen möglichst dauerhaften Halt zu geben. Man spricht auch von langem Frisurenhalt oder von einem langanhaltenden Haltegrad des Stylingmittels. Der Frisurenhalt wird im Wesentlichen durch die Art und Menge des eingesetzten festigenden Wirkstoffs bestimmt, wobei jedoch auch ein Einfluss der weiteren Bestandteile des Stylingmittels gegeben sein kann.

Neben einem langanhaltenden Haltegrad müssen Stylingmittel eine ganze Reihe weiterer Anforderungen erfüllen. Diese können grob in Eigenschaften am Haar, Eigenschaften der jeweiligen Formulierung, zum Beispiel Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit (tack) und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, sind als festigende Wirkstoffe bereits eine Vielzahl von synthetischen Polymeren entwickelt worden, die in Stylingmitteln zur Anwendung kommen. Die Polymere lassen sich in kationische, anionische, nichtionische und amphotere festigende Polymere unterteilen.

Die in kosmetischen Mitteln zur temporären Formgebung üblicherweise eingesetzten synthetischen Polymere werden aus entsprechenden synthetisch zugänglichen Monomeren hergestellt. Besagte Monomere werden aus fossilen Stoffen wie beispielsweise Erdöl durch Umwandlung zu den entsprechenden Polymerbausteinen u.a. unter Aufwand von Energie gewonnen. Da bereits die Monomere synthetisch hergestellt werden, handelt es sich bei den üblicherweise eingesetzten Polymere um vollsynthetische Polymere. Im Rahmen eines nachhaltigeren Umganges mit Rohstoffen und Energie bleibt es wünschenswert, für kosmetische Produkte nur solche kosmetischen Rohstoffe zu verwenden, die unter möglichst wenig Einsatz von Energie aus nachwachsenden Rohstoffen zugänglich sind. Eine Mengenreduktion oder gar ein Austausch besagter vollsynthetischer Polymere kann allerdings nur dann vorgenommen werden, wenn die Ersatzpolymere die für den Anwendungszweck gewünschten Eigenschaften hervorrufen und den keratinhaltigen Fasern einen ausreichenden, stabilen Halt der aufgeprägten Form geben.

Eine Aufgabe der vorliegenden Erfindung war es, kosmetische Mittel zur temporären Verformung keratinischer Fasern bereitzustellen, bei dem der Einsatz vollsynthetischer Polymere deutlich reduziert wird oder bei dem auf den Einsatz vollsynthetischer Polymere verzichtet wird und die Frisuren einen lang anhaltenden Haltegrad verleihen.

Diese Aufgabe wird gelöst durch ein kosmetisches Mittel zur temporären Umformung keratinischer Fasern, enthaltend:

- mindestens ein amphoteres Stärkederivat und
- mindestens eine Carboxymethylstärke,

gemäß Anspruch 1.

Es wurde im Rahmen der vorliegenden Erfindung überraschend festgestellt, dass die Kombination aus mindestens einem amphoteren Stärkederivat und mindestens einem Stärkephosphat in einem kosmetischen Mittel zur temporären Verformung von keratinischen Fasern, insbesondere von menschlichem Haar, zu einem unerwartet hohem Langzeithalt von Frisuren führt. In anderen Worten, die Haltbarkeit einer mittels eines erfindungsgemäßen Mittels zur temporärer Verformung hergestellten Frisur kann verlängert werden.

Andere üblicherweise geforderte Eigenschaften von kosmetischen Mitteln zur temporären Umformung keratinischer Fasern, wie geringe Klebrigkeit und gute Feuchtebeständigkeit, blieben dabei erhalten. Die beiden Stärkederivate basieren zudem auf einem natürlichen, nachwachsenden Rohstoff.

Die vorliegende Erfindung betrifft insbesondere kosmetische Stylingmittel wie Haargele, Haarwachse, Pasten, Lotionen, Emulsionen oder Clays. Die Produktform "Clay" bezeichnet hochviskose, wachsartige kosmetische Mittel, die unter anderem Tonverbindungen (zum Beispiel Kaolin) enthalten. Der Begriff keratinische Fasern umfasst erfindungsgemäß Pelze, Wolle und Federn, insbesondere aber menschliche Haare.

Als ersten erfindungswesentlichen Inhaltsstoff enthält das kosmetische Mittel mindestens ein amphoteres Stärkederivat, gemäß Formel (I) unten.

Amphotere Stärkederivate enthalten eine oder mehrere anionische Gruppen und eine oder mehrere kationische Gruppen. Anionische und kationische Gruppen können an dieselbe reaktive Stelle des Stärkemoleküls oder an verschiedene Stellen gebunden sein. Vorzugsweise sind sie an dieselbe reaktive Stelle des Stärkemoleküls gebunden. Anionische Gruppen können vom Carbonsäuretyp, Phosphattyp oder Sulfattyp sein und sind vorzugsweise vom Carbonsäuretyp. Die kationischen Gruppen können vom Typ des primären Amins, des sekundären Amins, des tertiären Amins oder des quaternären Amins sein.

Gemäß der Erfindung ist das amphotere Stärkederivat aus Formel (I) ausgewählt:

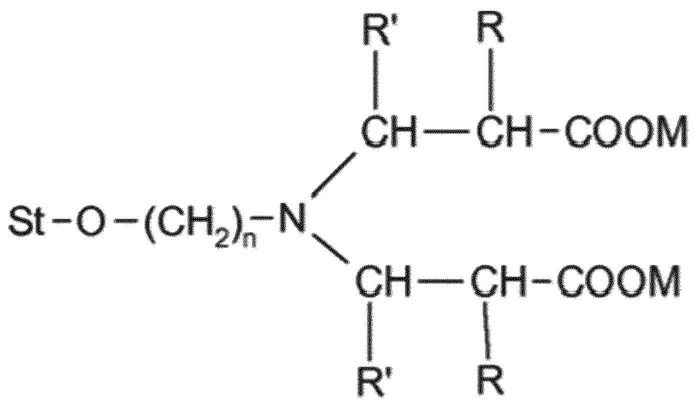

(I)

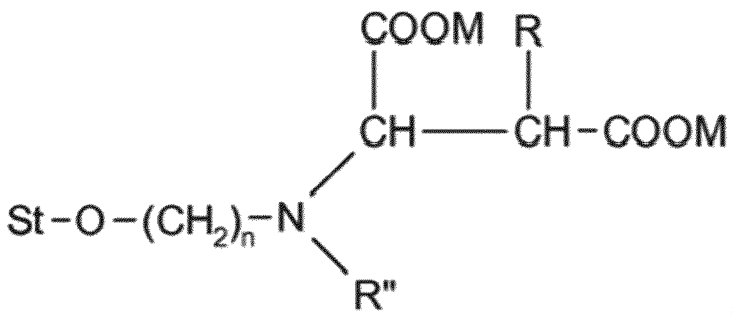

(II)

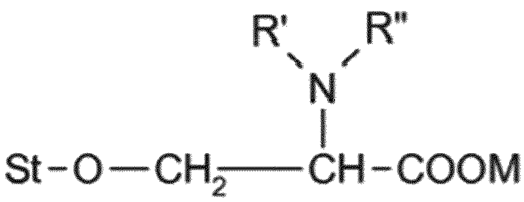

(III)

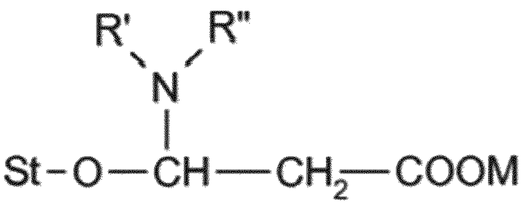

(IV)

ST-O steht für ein Stärkemolekül,

R ist gleich oder verschieden und steht für ein Wasserstoffatom oder ein Methylradikal,

R’ ist gleich oder verschieden und steht für ein Wasserstoffatom, ein Methylradikal oder eine - COOH-Gruppe,

n ist eine ganze Zahl gleich 2 oder 3,

M ist gleich oder verschieden und steht für ein Wasserstoffatom, ein Alkalimetall oder Erdalkalimetall wie Na, K, Li, $NH_4$, ein quaternäres Ammonium oder ein organisches Amin;

R" steht für ein Wasserstoffatom oder ein Alkylradikal mit 1 bis 18 Kohlenstoffatomen.

Insbesondere werden amphotere Stärkederivate eingesetzt, die mit 2-Chlorethylaminodipropionsäure modifiziert wurden. Dies sind die amphoteren Stärkederivate der Formel (I), in denen R, R', und M ein Wasserstoffatom darstellen und n gleich 2 is Weitere amphoteren Stärkederivate gemäß Formeln (II)-(IV) oben können zusätzlich angewendet werden.

In einer besonders bevorzugten Ausführungsform wird als amphotere Stärkederivat eine Kartoffelstärke eingesetzt, die mit 2-Chlorethylaminodipropionsäure modifiziert und mit NaOH neutralisiert wurde. Eine solche modifizierte Kartoffelstärke ist beispielsweise unter der Bezeichnung Structure® Solanace von Nouryon erhältlich.

Das kosmetische Mittel enthält das amphotere Stärkederivat vorzugsweise in einer Menge von 0,1 bis 10 Gew.-%, mehr bevorzugt von 0,25 bis 8 und ganz besonders bevorzugt von 0,4 bis 5 Gew.-%, jeweils bezogen auf das Gesamtgewicht des kosmetischen Mittels.

Als zweiten erfindungswesentlichen Inhaltsstoff enthält das kosmetische Mittel mindestens eine Carboxymethylstärke.

Carboxymethylstärke (CMS) ist ein Stärkeether, bei denen ein Teil der Hydroxygruppen als Ether mit einer Carboxymethylgruppe ($-CH_2COOH$) verknüpft sind. Zur Herstellung wird eine Stärke mit Lauge in Alkalistärke überführt und diese anschließend mit Chloressigsäure zur Carboxymethylstärke alkyliert. Überwiegend wird dabei Natronlauge eingesetzt, so dass die meisten im Handel verfügbaren Carboxymethylstärken Natriumsalze sind, welche sich hinsichtlich ihres Natriumgehaltes (ergo Carboxymethylierungsgrades) unterscheiden können.

Besonders bevorzugte Carboxymethylstärken sind Verbindungen der folgenden Formel

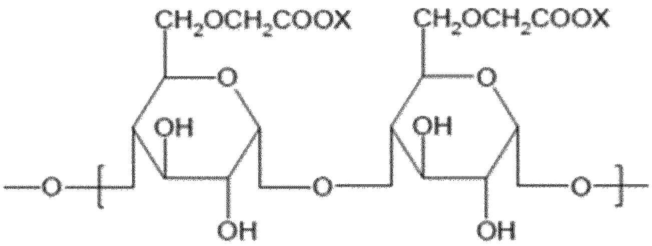

in der jedes X unabhängig voneinander kovalent oder nicht kovalent an die Carboxylgruppe gebunden ist und ein Wasserstoffatom, ein Alkali- oder Erdalkalimetall wie Na, K oder Li, $NH_4$, eine quaternäre Ammoniumverbindung oder ein organisches Amin wie zum Beispiel Mono-, Di- oder Triethanolamin bedeutet. Vorzugsweise ist X ein $Na^+$-Kation.

Die Carboxymethylstärke, kann teilweise oder vollständig vernetzt sein.

Geeignete Natriumsalze von Carboxymethylstärke (INCI-Bezeichnung: SODIUM CARBOXYMETHYL STARCH) werden beispielsweise unter der Bezeichnung "Beauté by Roquette® ST 118" von der Firma Roquette verkauft.

Die verwendeten Stärkemoleküle können aus einer pflanzlichen Quelle wie Getreide, Knollen, Wurzeln, Hülsenfrüchten und Früchten stammen. Vorzugsweise stammt die Stärke aus einer pflanzlichen Quelle, umfassend Mais, Erbse, Kartoffel, Süßkartoffel, Banane, Gerste, Weizen, Reis, Hafer, Sago, Tapioka und Sorghum. Vorzugsweise wird die Stärke aus Kartoffeln gewonnen.

Das kosmetische Mittel enthält die Carboxymethylstärke vorzugsweise in einer Menge von 0,1 bis 10 Gew.-%, mehr bevorzugt von 0,25 bis 8 und ganz besonders bevorzugt von 0,4 bis 5 Gew.-%, jeweils bezogen auf das Gesamtgewicht des kosmetischen Mittels.

In einer bevorzugten Ausführungsform kann das kosmetische Mittel mit weiteren, insbesondere filmbildenden, Polymeren formuliert sein. Das kosmetische Mittel kann weiterhin beispielsweise Acrylat(co)polymere, Polyurethanpolymeren oder Vinylpyrrolidon-haltige Homo- und Copolymere, wie PVP oder PVP/VA, enthalten.

Aufgrund ihrer kosmetischen Wirkung in Kombination mit den beiden Stärkederivaten umfassen die weiteren, filmbildende Polymere vorzugsweise Polyvinylpyrrolidone (INCI-Bezeichnung: PVP) und/oder Vinylpyrrolidon/Vinylacetat-Copolymere (INCI-Bezeichnung VP/VA Copolymer). Durch den Zusatz weiterer, filmbildender Polymere, insbesondere der vorgenannten Polyvinylpyrrolidone und Vinylpyrrolidon/Vinylacetat-Copolymere werden vernehmlich die Halteeigenschaften aber auch die Applikationseigenschaften der kosmetischen Mittel vorteilhaft beeinflusst. Der Gewichtsanteil dieser weiteren Polymere ist vorzugsweise auf Mengen zwischen 1 und 15 Gew.-%, bezogen auf das Gesamtgewicht des kosmetischen Mittels, beschränkt. Bevorzugte kosmetische Mittel sind daher dadurch gekennzeichnet, dass sie bezogen auf ihr Gesamtgewicht weiterhin 1 bis 15 Gew.-% Polyvinylpyrrolidon und/oder ein Vinylpyrrolidon/Vinylacetat-Copolymer, vorzugsweise Polyvinylpyrrolidon enthalten. Besonders bevorzugte kosmetische Mittel weisen einen Gewichtsanteil des Polyvinylpyrrolidons und/oder des Vinylpyrrolidon/Vinylacetat-Copolymers am Gesamtgewicht des kosmetischen Mittels von 2 bis 10 Gew.-%, vorzugsweise von 3 bis 10 Gew.-% auf.

Das kosmetische Mittel kann als weitere Komponente ein natürliches oder synthetisches Wachs, welches einen

Schmelzpunkt von über 37°C aufweist, enthalten. Das kosmetische Mittel kann das Wachs in einer Gesamtmenge von 1 bis 30 Gew.-%, bevorzugt 2 bis 25 Gew.-% und mehr bevorzugt 2,5 bis 20 Gew.-%, bezogen auf das Gesamtgewicht des kosmetischen Mittels, enthalten

Das kosmetische Mittel enthält vorzugsweise einen Emulgator. Als Emulgatoren kommen anionische, kationische, nichtionische und ampholytische oberflächenaktive Verbindungen in Frage, die für die Anwendung am menschlichen Körper geeignet sind. Die ampholytischen oberflächenaktiven Verbindungen umfassen zwitterionische oberflächenaktive Verbindungen und Ampholyte. Bevorzugt sind nichtionische Emulgatoren.

Als nichtionische Emulgatoren sind insbesondere Anlagerungsprodukte von Ethylenoxid an lineare Fettalkohole, an Fettsäuren, an Fettsäurealkanolamide, an Fettsäuremonoglyceride, an Sorbitanfettsäuremonoester, an Fettsäureglyceride, an Methylglucosidmonofettsäureester, an Polydimethylsiloxane und Gemische davon verwendbar.

Besonders bevorzugte nichtionische Tenside umfassen die Anlagerungsprodukte von 5 bis 60 Mol Ethylenoxid an Rizinusöl oder gehärtetes (hydriertem) Rizinusöl. Bevorzugt sind Ethylenoxid-Anlagerungsprodukte von gehärtetem Rizinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil oder PEG-40 Hydrogenated Castor Oil. Bevorzugt ist die Verwendung von PEG-40 Hydrogenated Castor Oil.

Das kosmetische Mittel kann den Emulgator in einer Gesamtmenge von 0,1 bis 2 Gew.-%, bevorzugt 0,25 bis 1,5 Gew.-%, noch bevorzugt 0,5 bis 1 Gew.-%, bezogen auf das Gesamtgewicht des kosmetischen Mittels, enthalten.

Als weitere geeignete Hilfs- und Zusatzstoffe, die in dem kosmetischen Mittel enthalten sein können, sind insbesondere Pflegekomponenten, wie beispielsweise Öle, Proteinhydrolysate, Vitamine, Provitamine, Vitaminvorstufen, zu nennen.

Zur Einstellung des pH kann das kosmetische Mittel weiterhin pH-Stellmittel enthalten. Beispiele von in kosmetischen Mitteln verwendeten pH-Stellmitteln sind primäre Aminoalkohole wie Aminomethyl Propanol (INCI), das im Handel beispielsweise unter der Bezeichnung AMP-ULTRA® PC erhältlich ist, beispielsweise AMP-ULTRA® PC 2000.

Die Mittel können weiterhin kosmetisch akzeptable Konservierungsmittel enthalten. Ein Beispiel eines bevorzugt verwendbaren Konservierungsmittels ist 2-Phenoxyethanol.

Das kosmetische Mittel der vorliegenden Erfindung kann in den für die temporäre Umformung von Haaren üblichen Formen konfektioniert sein, beispielsweise als Wachs, Paste, Lotion, Emulsion oder Clay. Bevorzugt werden die kosmetischen Mittel in Dosen oder Tiegeln angeboten.

Das kosmetische Mittel ist vorzugsweise ein Haargel. Haargel ist im Sinne der vorliegenden Erfindung umfasst einen Frisurenfestiger in Gel-Form auf Wasser- oder Wasser-/Alkohol-Basis, der verwendet wird, um die Frisur zu gestalten (Styling).

Bevorzugte kosmetische Mittel auf Wasser- oder Wasser-/Alkohol-Basis enthalten, bezogen auf ihr Gesamtgewicht, mindestens 20 Gew.-%, vorzugsweise mindestens 40 Gew.-% und insbesondere mindestens 70 Gew.-% Wasser. Bevorzugte kosmetische Mittel auf Wasser-/Alkohol-Basis enthalten ferner, bezogen auf ihr Gesamtgewicht, mindestens 2 Gew.-%, vorzugsweise mindestens 5 Gew.-% und insbesondere mindestens 7,5 Gew.-% Ethanol.

Die Bereitstellung des kosmetischen Mittels in Form eines Mousses, Schaums oder Sprays kann sowohl ohne Zusatz eines Treibmittels, beispielsweise mittels einer mechanischen Pump-, Schäum- oder Sprühvorrichtung aber auch unter Einsatz eines Treibmittels (z.B. Aerosolspray) erfolgen. Entsprechende kosmetische Mittel umfassen dann weiterhin mindestens ein Treibmittel.

Die vorliegende Erfindung betrifft auch die kosmetische Verwendung der erfindungsgemäßen kosmetischen Mitteln zur temporären Umformung von keratinischen Fasern, insbesondere von menschlichen Haaren, die Verwendung der erfindungsgemäßen kosmetischen Mitteln zur temporären Umformung von keratinischen Fasern, um einer Frisur langanhaltenden Halt zu verleihen sowie ein Verfahren zur temporären Verformung von keratinischen Fasern, insbesondere menschlichen Haaren, bei dem das kosmetische Mittel auf keratinische Fasern aufgebracht wird.

Beispiele

Es wurde folgende Haargele hergestellt:

| **Komponente/Rohstoff** | **INCI-Bezeichnung** | **V1** | **V2** | **E1** |
|---|---|---|---|---|
| Structure® Solanace | Potato Starch Modified | 0,5 | -- | 0,5 |
| Beauté by Roquette® ST 118 | Sodium Carboxymethyl Starch | -- | 0,5 | 0,5 |
| Wasser | | 91,5 | 91,5 | 91 |
| Ethanol | | 8 | 8 | 8 |
| **Gesamt** | | 100 | 100 | 100 |

Die Mengenangaben in der Tabelle sind in Gew.-% des jeweiligen Rohstoffs, bezogen auf das gesamte kosmetische Mittel, angegeben.

Der Langzeithalt des Ausführungsbeispiels E1 und der Vergleichsbeispiele V1 und V2 wurde gemessen.

Die Mittel wurden mittels einer Long Lasting Hold Messung hinsichtlich ihrer formgebenden Eigenschaften überprüft. Hierzu wurden standardisierte Haarsträhnen der Fa. Kerling (Art. Nr. 826500) des Haartyps "European Natural, Farbe 6/0) von einer Länge ($L_{max}$) von 220 mm und einem Gewicht von 3,0 g eingesetzt. Zur Vorbereitung wurden die Strähnen mit einer 12,5 Gew.- %igen Natriumlaurethsulfat-Lösung gewaschen. Die Haarsträhnen wurden über Nacht in einem Trockenofen bei 45 °C getrocknet.

Die Haare wurden 20 min in lauwarmen Wasser eingeweicht und dann auf ca. 50% Restfeuchte im Haare abgetupft,

750 mg der zu untersuchenden Zusammensetzung wurden auf jede der Haarsträhnen aufgebracht und einmassiert. Die Haarsträhnen wurden in eine Teflonschiene eingelegt, mittels einer Stahlrolle geglättet und über Nacht bei 21 °C und 80% Luftfeuchtigkeit getrocknet.

Die Haarsträhnen wurden nachfolgend an ihrem einen Ende in eine Haltevorrichtung eingespannt, und für eine Dauer von sechs Stunden bei 21 °C und 85% relativer Luftfeuchte gelagert. Zur Berechnung des Long Lasting Hold (LLH) wurden die aus der Haltevorrichtung herausragende Strähnenlänge vor ($L_0$) und nach ($L_t$) der Lagerung gemessen.

Der Long Lasting Hold ist ein Maß für die zeitliche Längenänderung einer mittels eines Haarverformungsmittels fixierten Haarsträhne. Je höher der LLH-Wert, desto geringer die Längenänderung der Haarsträhne unter Einfluss von Luftfeuchtigkeit in einer bestimmten Zeitdauer und desto besser der Haltegrad des Haarverformungsmittels.

Der Long Lasting Hold wurde gemäß folgender Formel berechnet.

$$LLH = 1 - (L_t - L_0/L_{max})$$

| | V1 | V2 | E1 | (V1 +V2) |
|---|---|---|---|---|
| LLH | 8,23 | 5,71 | 17,31 | 13,94 |

Das erfindungsgemäße kosmetische Mittel E1 zeigte demnach einen deutlich überadditiven, synergistischen Effekt in Bezug auf den Langzeithalt.

Ferner wurde das folgende Haargel E2 hergestellt:

| Komponente/Rohstoff | INCI-Bezeichnung | E2 |
|---|---|---|
| Structure® Solanace | Potato Starch Modified | 0,5 |
| Beauté by Roquette® ST 118 | Sodium Carboxymethyl Starch | 0,5 |
| Luviskol® VA 64 P | VP/VA Copolymer | 5 |
| Parfum | | 0,25 |
| Wasser | | 85,75 |
| Ethanol | | 8 |
| **Gesamt** | | 100 |

**Patentansprüche**

1. Kosmetisches Mittel zur temporären Umformung keratinischer Fasern, enthaltend:

   - mindestens ein amphoteres Stärkederivat und
   - mindestens eine Carboxymethylstärke,

   **dadurch gekennzeichnet, dass** das amphotere Stärkederivat ausgewählt ist aus Verbindungen der Formel (I)

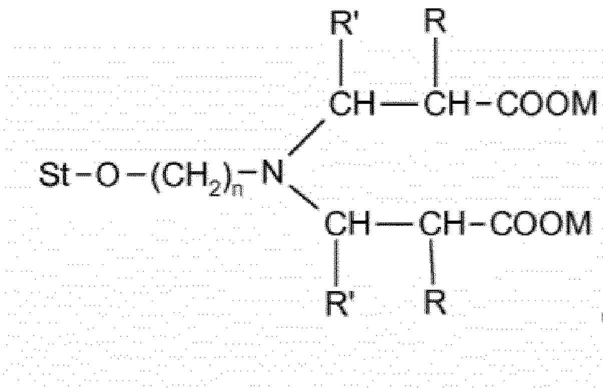

(I)

wobei ST-O für ein Stärkemolekül steht,
R gleich oder verschieden ist und für ein Wasserstoffatom oder ein Methylradikal steht.
R' gleich oder verschieden ist und für ein Wasserstoffatom, ein Methylradikal oder eine -COOH-Gruppe steht,
n eine ganze Zahl gleich 2 oder 3 ist,
M gleich oder verschieden ist und für ein Wasserstoffatom, ein Alkalimetall oder Erdalkalimetall wie Na, K, Li, $NH_4$, ein quaternäres Ammonium oder ein organisches Amin steht.

2. Kosmetisches Mittel nach Anspruch 1, **dadurch gekennzeichnet, dass** R, R' und M ein Wasserstoffatom darstellen und n gleich 2 ist.

3. Kosmetisches Mittel nach einem der Ansprüche 1 oder 2, **dadurch gekennzeichnet, dass** die Menge an amphoterem Stärkederivat von 0,1 bis 10 Gew.-%, mehr bevorzugt von 0,25 bis 8 und ganz besonders bevorzugt von 0,5 bis 5 Gew.-%, jeweils bezogen auf das Gesamtgewicht des kosmetischen Mittels, beträgt.

4. Kosmetisches Mittel nach einem der Ansprüche 1 bis 3, **dadurch gekennzeichnet, dass** die mindestens eine Carboxymethylstärke ein Natriumsalz von Carboxymethylstärke (INCI: Sodium Carboxymethyl Starch) umfasst.

5. Kosmetisches Mittel nach einem der Ansprüche 1 bis 4, **dadurch gekennzeichnet, dass** die Menge an Carboxymethylstärke von 0,1 bis 10 Gew.-%, mehr bevorzugt von 0,25 bis 8 und ganz besonders bevorzugt von 0,5 bis 5 Gew.-%, jeweils bezogen auf das Gesamtgewicht des kosmetischen Mittels, beträgt.

6. Kosmetisches Mittel gemäß einem der vorhergehenden Ansprüche, **dadurch gekennzeichnet, dass** das Mittel weiterhin Polyvinylpyrrolidon und/oder ein Vinylpyrrolidon/Vinylacetat-Copolymer enthält.

7. Verwendung eines kosmetischen Mittels gemäß einem der vorhergehenden Ansprüche zur temporären Verformung von keratinischen Fasern, insbesondere menschlichen Haaren, welches vorzugsweise als Haargel vorliegt.

8. Verwendung eines kosmetischen Mittels gemäß Anspruch 7, um einer Frisur langanhaltenden Halt zu verleihen.

9. Verfahren zur temporären Umformung keratinischer Fasern, insbesondere menschlicher Haare, **dadurch gekennzeichnet, dass** ein kosmetisches Mittel gemäß einem der Ansprüche 1-6 auf die keratinischen Fasern aufgebracht wird.

## Claims

1. Cosmetic composition for the temporary reshaping of keratinous fibers, containing:

   - at least one amphoteric starch derivative and
   - at least one carboxymethyl starch,

   **characterized in that** the amphoteric starch derivative is selected from compounds of formula (I)

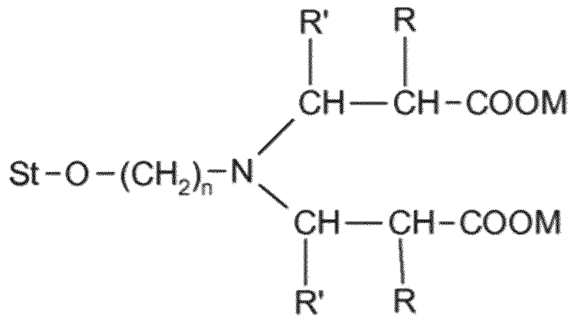

(I)

where ST-O stands for a starch molecule,
R is the same or different and represents a hydrogen atom or a methyl radical.
R' is the same or different and represents a hydrogen atom, a methyl radical or a- COOH group,
n is an integer equal to 2 or 3,
M is the same or different and represents a hydrogen atom, an alkali metal or alkaline earth metal such as Na, K, Li, $NH_4$ , a quaternary ammonium or an organic amine.

2. Cosmetic composition according to claim 1, **characterized in that** R, R' and M represent a hydrogen atom and n is equal to 2.

3. Cosmetic composition according to one of claims 1 or 2, **characterized in that** the amount of amphoteric starch derivative is from 0.1 to 10% by weight, more preferably from 0.25 to 8 and very particularly preferably from 0.5 to 5% by weight, in each case based on the total weight of the cosmetic composition.

4. Cosmetic composition according to any one of claims 1 to 3, **characterized in that** the at least one carboxymethyl starch comprises a sodium salt of carboxymethyl starch (INCI: Sodium Carboxymethyl Starch).

5. Cosmetic composition according to any one of claims 1 to 4, **characterized in that** the amount of carboxymethyl starch is from 0.1 to 10% by weight, more preferably from 0.25 to 8 and very particularly preferably from 0.5 to 5% by weight, in each case based on the total weight of the cosmetic composition.

6. Cosmetic composition according to any one of the preceding claims, **characterized in that** the composition further comprises polyvinylpyrrolidone and/or a vinylpyrrolidone/vinyl acetate copolymer.

7. Use of a cosmetic composition according to one of the preceding claims for the temporary deformation of keratinous fibers, in particular human hair, which is preferably present as a hair gel.

8. Use of a cosmetic composition according to claim 7 to impart long-lasting hold to a hairstyle.

9. Process for the temporary reshaping of keratinous fibres, in particular human hair, **characterized in that** a cosmetic agent according to one of claims 1-6 is applied to the keratinous fibres.

## Revendications

1. Composition cosmétique pour le remodelage temporaire des fibres kératiniques, contenant

   - au moins un dérivé amphotère de l'amidon, et
   - au moins un carboxyméthylamidon,

   **caractérisé en ce que** le dérivé d'amidon amphotère est choisi parmi les composés de formule (I)

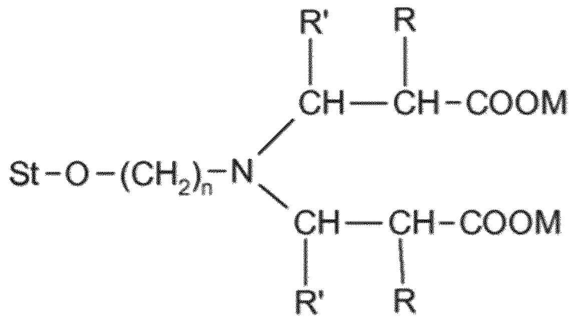

(I)

où ST-O représente une molécule d'amidon,
R est identique ou différent et représente un atome d'hydrogène ou un radical méthyle.
R' est identique ou différent et représente un atome d'hydrogène, un radical méthyle ou un groupe- COOH,
n est un nombre entier égal à 2 ou 3,
M est identique ou différent et représente un atome d'hydrogène, un métal alcalin ou un métal alcalino-terreux tel que Na, K, Li, $NH_4$ , un ammonium quaternaire ou une amine organique.

2. Composition cosmétique selon la revendication 1, **caractérisée en ce que** R, R' et M représentent un atome d'hydrogène et n est égal à 2.

3. Composition cosmétique selon l'une des revendications 1 ou 2, **caractérisée en ce que** la quantité de dérivé d'amidon amphotère est de 0,1 à 10 % en poids, plus préférentiellement de 0,25 à 8 et tout particulièrement de 0,5 à 5 % en poids, respectivement par rapport au poids total de la composition cosmétique.

4. Composition cosmétique selon l'une des revendications 1 à 3, **caractérisée en ce que** ledit au moins un carboxyméthylamidon comprend un sel de sodium de carboxyméthylamidon (INCI : Sodium Carboxymethyl Starch).

5. Composition cosmétique selon l'une des revendications 1 à 4, **caractérisée en ce que** la quantité de carboxyméthylamidon est de 0,1 à 10 % en poids, plus préférentiellement de 0,25 à 8 et tout particulièrement de 0,5 à 5 % en poids, respectivement par rapport au poids total de la composition cosmétique.

6. Composition cosmétique selon l'une quelconque des revendications précédentes, **caractérisée en ce que** la composition contient en outre de la polyvinylpyrrolidone et/ou un copolymère de vinylpyrrolidone/acétate de vinyle.

7. Utilisation d'une composition cosmétique selon l'une quelconque des revendications précédentes pour la mise en forme temporaire des fibres kératiniques, notamment des cheveux humains, qui se présente de préférence sous forme de gel capillaire.

8. Utilisation d'une composition cosmétique selon la revendication 7 pour conférer une tenue longue durée à une coiffure.

9. Procédé de transformation temporaire de fibres kératiniques, notamment de cheveux humains, **caractérisé en ce qu'**on applique sur les fibres kératiniques une composition cosmétique selon l'une quelconque des revendications 1 à 6.